# EUROPEAN PATENT APPLICATION

(11) **EP 4 324 841 A1**
(43) Date of publication of application: **21.02.2024**
(21) Application number: 22788487.1
(22) Date of filing: 15.04.2022
(51) Int. Cl.: C07K 7/08, C07K 7/06, C07K 14/00, A61K 38/00, A61P 35/00

(54) **CELL-PENETRATING PEPTIDE, ANTI-CANCER PEPTIDE, AND PHARMACEUTICAL COMPOSITION FOR PREVENTING OR TREATING CANCER, COMPRISING SAME**

(30) Priority: 15.04.2021 KR 20210049443
(71) Applicant: Onecuregen Co., Ltd., Daejeon 34141 (KR); Icure BNP Co., Ltd., Chungcheongbuk-do 27643 (KR); Korea Research Institute of Bioscience and Biotechnology, Daejeon 34141 (KR)
(72) Inventor: CHANG, Kwan Young, Seoul 07649 (KR); WON, Mi Sun, Daejeon 34141 (KR); HWANG, In Ah, Daejeon 34141 (KR); KIM, Bo Kyung, Daejeon 34141 (KR); KIM, Kyeong Soo, Daejeon 34141 (KR); KANG, Seo Hee, Seoul 03328 (KR); HAN, Song Yee, Incheon 21404 (KR)
(74) Representative: Frenkel, Matthias Alexander
(86) International application number: PCT/KR2022/005446
(87) International publication number: WO 2022/220632

(57) **Abstract**

The present disclosure relates to a cell-penetrating peptide, an anticancer peptide, and a pharmaceutical composition for preventing or treating cancer including the same. The cell-penetrating peptide of the present disclosure can further improve a cancer cell growth inhibitory effect of anticancer peptides, especially cancer therapeutic peptides with improved anticancer activity by cyclic structures, and thus can be effectively applied to the prevention or treatment of various cancers.

## Description

### [Technical Field]

The present disclosure relates to a cell-penetrating peptide, an anticancer peptide, and a pharmaceutical composition for preventing or treating cancer including the same. Particularly, the present disclosure relates to a composition and a method for treating cancer using an anticancer peptide for inhibiting cancer growth and metastasis and a cell-penetrating peptide for enhancing the cell penetration rate of the peptide by inhibiting the interaction between VGLL1 and TEAD4.

### [Background Art]

Cancer has a high death rate worldwide and is the most common death cause next to cardiovascular disease in the Western society. In particular, due to the aging of the population, the generalization of intake of high-fat diet due to westernization of dietary life, a rapid increase in environmental pollutants, an increase in volume of drink, and the like, colon cancer, breast cancer, prostate cancer, etc. are continuously increasing. In this situation, the creation of anticancer substances has been urgently required by enabling the early prevention and treatment of cancer to enhance human health, improve the quality of health life, and contribute to human health care promotion.

In many cancers, the functions of cancer-related genes are regulated by activation of various signaling systems through cytokines. Transforming growth factor-β (TGF-β) is involved in various functions such as cell growth inhibition, apoptosis, differentiation, and epithelial-mesenchymal transition (EMT) by regulating signaling pathways of SMAD, Rho, PP2A, and cancer-related genes. Many studies have been conducted on various mechanisms and broad roles of TGF-β as a pleiotropic factor in animal models and patients.

A vestigial-like (VGLL) gene, which has structural similarity to a vestigial (vg) gene, a Drosophila transcription factor coactivator, acts as a cofactor for TEA domain transcription factors (TEADs) that bind to a TEA region. VGLL1 has high structural homology with a Yes-associated protein (YAP) and a transcription coactivator with a PDZ-binding motif (TAZ) to form a VGLL1-TEAD4 complex. In addition, the transcription of VGLL1 is regulated through a signaling mechanism in PI3K-AKT-β-catenin, and the VGLL1-TEAD4 complex has been reported to be involved in the growth and metastasis of stomach cancer by increasing the expression of Matrix metallopeptidase 9 (MMP9). However, nothing is known about the specific actions of VGLL1 in relation to a series of growth and metastasis of cancer cells, and there have been almost no reports on cancer cell growth or cancer metastasis related to enhanced expression of VGLL1.

On the other hand, cell-penetrating peptides (CPPs) are peptides consisting of about 10 to 60 amino acids, and are penetrated by inducing cell membrane penetration by a protein-transduction domain or membrane-translocating sequence. Unlike a general endocytosis pathway for foreign substances, the CPP is known to move into cells without damaging the cell membrane, and also to deliver DNA or proteins that are known not to pass through the cell membrane into cells.

Accordingly, research has been conducted to develop peptide-based anticancer agents that may selectively eliminate only cancer cells using cell-penetrating peptides, but peptide-based anticancer agents with significantly improved anticancer effects through the application of the cell-penetrating peptides have not yet been developed.

Therefore, the present inventors conducted research to develop anticancer peptides with improved effects, cell-penetrating peptides, and peptide-based anticancer agents using these cell-penetrating peptides, and then completed the present disclosure.

### [Disclosure]

### [Technical Problem]

An object of the present disclosure is to provide a peptide having a length of 20 amino acids or less, including an amino acid sequence in which an amino acid sequence represented by the following Formula I is linked to an N-terminus or C-terminus of an amino acid sequence represented by SEQ ID NO: 7:

[Formula I] Xaa₁-Xaa₂-Xaa₃-Xaa₄-Xaa₅ (SEQ ID NO: 1)

in Formula, Xaa₁ is an amino acid selected from the group consisting of Arg, Lys, Ala, Val, Ile, Leu, Met, Phe, Tyr and Trp, Xaa₂ is an amino acid selected from the group consisting of Arg, Lys and His, Xaa₃ is an amino acid of Arg or Leu, Xaa₄ is an amino acid selected from the group consisting of Arg, Lys, His, Asp, Glu, Ser, Thr, Asn and Gln, and Xaa₅ is an amino acid selected from the group consisting of Arg, Lys and Val.

Another object of the present disclosure is to provide a cell penetrating composition including the peptide.

Yet another object of the present disclosure is to provide a peptide including an amino acid sequence represented by SEQ ID NO: 2 and having a length of 35 amino acids or less.

Yet another object of the present disclosure is to provide a pharmaceutical composition for preventing or treating cancer including the peptide.

### [Technical Solution]

An aspect of the present disclosure provides a peptide having a length of 20 amino acids or less, including an amino acid sequence in which an amino acid sequence represented by the following Formula I is linked to an N-terminus or C-terminus of an amino acid sequence represented by SEQ ID NO: 7:

[Formula I] Xaa₁-Xaa₂-Xaa₃-Xaa₄-Xaa₅

in Formula,
Xaa₁ is an amino acid selected from the group consisting of Arg, Lys, Ala, Val, Ile, Leu, Met, Phe, Tyr and Trp,
Xaa₂ is an amino acid selected from the group consisting of Arg, Lys and His,
Xaa₃ is an amino acid of Arg or Leu,
Xaa₄ is an amino acid selected from the group consisting of Arg, Lys, His, Asp, Glu, Ser, Thr, Asn and Gln, and
Xaa₅ is an amino acid selected from the group consisting of Arg, Lys and Val.

According to one embodiment of the present disclosure, 1 to 10 arginines (Arg) or lysines (Lys) may be further linked to the N-terminus or C-terminus of the peptide.

According to one embodiment of the present disclosure, the peptide may consist of an amino acid sequence represented by SEQ ID NO: 12 or SEQ ID NO: 25.

Another aspect of the present disclosure provides a cell penetrating composition including the peptide.

Yet another aspect of the present disclosure provides a peptide including an amino acid sequence represented by SEQ ID NO: 2 and having a length of 35 amino acids or less.

According to one embodiment of the present disclosure, the peptide may entirely or partially include a cyclic structure.

According to one embodiment of the present disclosure, the cyclic structure includes cysteine (Cys)-cysteine (Cys) disulfide bonds or linkers.

According to one embodiment of the present disclosure, the peptide may consist of any one amino acid sequence selected from the group consisting of SEQ ID NO: 3, SEQ ID NO: 27, SEQ ID NO: 29, SEQ ID NO: 31, SEQ ID NO: 33, SEQ ID NO: 40, SEQ ID NO: 41, SEQ ID NO: 42, SEQ ID NO: 44, SEQ ID NO: 46, SEQ ID NO: 48 and SEQ ID NO: 50.

Yet another aspect of the present disclosure provides a pharmaceutical composition for preventing or treating cancer including the peptide.

According to one embodiment of the present disclosure, the peptide may be a fusion peptide that is linked to a peptide having a length of 20 amino acids or less, including an amino acid sequence in which an amino acid sequence represented by the following Formula I is linked to an N-terminus or C-terminus of an amino acid sequence represented by SEQ ID NO: 7:

[Formula I] Xaa₁-Xaa₂-Xaa₃-Xaa₄-Xaa₅

in Formula,
Xaa₁ is an amino acid selected from the group consisting of Arg, Lys, Ala, Val, Ile, Leu, Met, Phe, Tyr and Trp,
Xaa₂ is an amino acid selected from the group consisting of Arg, Lys and His,
Xaa₃ is an amino acid of Arg or Leu,
Xaa₄ is an amino acid selected from the group consisting of Arg, Lys, His, Asp, Glu, Ser, Thr, Asn and Gln, and
Xaa₅ is an amino acid selected from the group consisting of Arg, Lys and Val.

According to one embodiment of the present disclosure, the peptide may consist of any one amino acid sequence selected from the group consisting of SEQ ID NO: 27, SEQ ID NO: 29, SEQ ID NO: 31, SEQ ID NO: 33, SEQ ID NO: 40, SEQ ID NO: 41, SEQ ID NO: 42, SEQ ID NO: 44, SEQ ID NO: 46, SEQ ID NO: 48 and SEQ ID NO: 50.

According to one embodiment of the present disclosure, the cancer may be any one cancer selected from the group consisting of stomach cancer, liver cancer, colon cancer, lung cancer, breast cancer, pancreatic cancer, head and neck squamous cell carcinoma, esophageal cancer, ovarian cancer, cervical cancer, endometrial cancer, mesothelioma, melanoma, sarcoma, osteosarcoma, liposarcoma, thyroid cancer, desmoid tumor, and blood cancer.

### [Advantageous Effects]

According to the cell-penetrating peptide, the anticancer peptide, and the pharmaceutical composition for preventing or treating cancer including the same, the cell-penetrating peptide of the present disclosure can further improve a cancer cell growth inhibitory effect of anticancer peptides, especially anticancer peptides with improved anticancer activity by cyclic structures, and thus can be effectively applied to the prevention or treatment of various cancers.

### [Description of Drawings]

FIG. 1 is a graph showing a cancer cell growth inhibitory effect by treatment with a VGLL1S84 anticancer peptide fragment linked with a TAT cell-penetrating peptide.
FIGS. 2 and 3 are diagrams comparing the cell permeability of a TAT cell-penetrating peptide and novel OCP cell-penetrating peptides on cancer cells.
FIG. 4 is a diagram comparing the cell permeability of a TAT cell-penetrating peptide and novel OCP cell-penetrating peptides on normal cells.
FIG. 5 is a graph showing investigating growth inhibitory effects of the novel cell-penetrating peptides and anticancer peptide fragments in Table 3 on a stomach cancer cell line (NUGC3).
FIG. 6 is a diagram comparing the growth inhibitory efficacy of cancer therapeutic peptides TAT-VGLLS84 and CCP5-VGLLS84 on various cancer cells.
FIG. 7 is a diagram showing a correlation between the lengths and the cancer cell growth inhibitory effect of an anticancer peptide in a CCP9-VGLLS84 structure.
FIG. 8 is a diagram showing a correlation between a cyclic shape of a cancer therapeutic peptide OCP9-VGLLS84 and stomach cancer cell (NUGC3) growth inhibitory efficacy depending on a concentration.
FIG. 9 is a graph showing results of evaluating cytotoxicity of cancer therapeutic peptide fragments on normal cell lines WI38 (A) and CCD39 (B).
FIG. 10 is a diagram showing a stomach cancer cell (AGS) migration inhibitory effect of a cancer therapeutic peptide fragment OCP9-VGLL1S84.
FIG. 11 is a graph and a diagram showing *in vivo* antitumor effects of OCP5-VGLL1S84-7C and OCP5-VGLL1S84-15C.
FIG. 12 is a graph and a diagram showing changes in tumor volume and tumor weight *in vivo* animal experiments using OCP5-VGLL1S84-7C and OCP5-VGLL1S84-15C.
FIG. 13 is a graph and a diagram showing decreases in MMP9 expression in tumors in an *in vivo* animal experiment using OCP5-VGLL 1 S84-7C.
FIG. 14 is a diagram showing the growth inhibitory efficacy of OCP5-VGLL1S84-7C on various cancer cells.

### [Best mode for carrying out the invention]

An aspect of the present disclosure provides a peptide having a length of 20 amino acids or less, including an amino acid sequence in which an amino acid sequence represented by the following Formula I is linked to an N-terminus or C-terminus of an amino acid sequence represented by SEQ ID NO: 7:

[Formula I] Xaa₁-Xaa₂-Xaa₃-Xaa₄-Xaa₅

in Formula, Xaa₁ is an amino acid selected from the group consisting of Arg, Lys, Ala, Val, Ile, Leu, Met, Phe, Tyr and Trp, Xaa₂ is an amino acid selected from the group consisting of Arg, Lys and His, Xaa₃ is an amino acid of Arg or Leu, Xaa₄ is an amino acid selected from the group consisting of Arg, Lys, His, Asp, Glu, Ser, Thr, Asn and Gln, and Xaa₅ is an amino acid selected from the group consisting of Arg, Lys and Val.

As used herein, the term "peptide" refers to a linear molecule formed by binding amino acid residues by peptide bonds, and may consist of 6 to 20 amino acid residues, preferably 10 to 16 amino acid residues, and most preferably 12 amino acid residues.

The peptide including the amino acid sequence represented by Formula I of the present disclosure may enhance the therapeutic effect of an anticancer peptide and thus may be effectively used to improve an anticancer effect of the anticancer peptide.

According to one embodiment of the present disclosure, 1 to 10 arginine (Arg) or lysine (Lys) may be further linked to the N-terminus or C-terminus of the peptide.

When at least one arginine or lysine is linked to the N-terminus or C-terminus of the peptide including the amino acid sequence represented by Formula I of the present disclosure, the hydrophilic activity may increase to further improve the anticancer effect of the anticancer peptide. The number of linked arginines or lysines may be 1 to 10, preferably 2 to 7, and most preferably 3 to 5.

According to one embodiment of the present disclosure, the peptide may consist of an amino acid sequence represented by SEQ ID NO: 12 or SEQ ID NO: 25.

Another aspect of the present disclosure provides a cell penetrating composition including the peptide.

As used herein, the term "cell-penetrating peptide (CPP)" refers to a peptide having the ability capable of delivering a cargo to be delivered, for example, an anticancer peptide, into cells, such as cancer cells *in vitro* and/or *in vivo,* and the cell-penetrating peptide itself is a peptide having an amino acid sequence capable of penetrating the cell membrane of the phospholipid bilayer.

As used herein, the term "cargo" refers to a chemical substance, a small molecule, a peptide, a nucleic acid, etc. that may be linked to a peptide including the amino acid sequence represented by Formula I, which acts as the cell-penetrating peptide, to be delivered into the cell.

In the present disclosure, there are various substances capable of binding to the terminus of the peptide including the amino acid sequence represented by Formula I, that is, corresponding to the cargo, and the substances include, for example, proteins (polypeptides), nucleic acids (polynucleotides), chemical substances (drugs), etc., but are not limited thereto.

For example, the substances may be drugs, contrast agents (e.g., a T1 contrast agent, a T2 contrast agent such as a superparamagnetic material, a radioactive isotope, etc.), fluorescent markers, dyes, etc., but are not limited thereto. The polypeptide corresponding to the cargo is a polymer of amino acids consisting of two or more residues and may include peptides and proteins. These polypeptides may be, for example, proteins (e.g., SV40 large T antigen and telomerase) involved in cell immortality, anti-apoptotic proteins (e.g., mutant p53 and BclxL), antibodies, oncogenes (e.g., ras, myc, HPV E6/E7, and adenovirus Ela), cell cycle regulatory proteins (e.g., cyclins and cyclin-dependent phosphorylase), or enzymes (e.g., green fluorescent protein, beta-galactosidase, and chloramphenicol acetyltransferase), but are not limited thereto. In addition, the nucleic acid may be, for example, RNA, DNA, or cDNA, and the sequence of the nucleic acid may be a coding region sequence or a non-coding region sequence (e.g., antisense oligonucleotide or siRNA). The nucleotides as the nucleic acid cargo may be standard nucleotides (e.g., adenosine, cytosine, guanine, thymine, inosine, and uracil) or analogs (e.g., phosphorothioate nucleotides). For example, the nucleic acid cargo may be an antisense sequence consisting of phosphorothioate nucleotides or RNAi.

Meanwhile, when the peptide including the amino acid sequence represented by Formula I to which the cargo is bound is given the opportunity to contact the cell membrane *in vitro* or *in vivo,* the cargo bound to the peptide is delivered into the cell.

Yet another aspect of the present disclosure provides a peptide including an amino acid sequence represented by SEQ ID NO: 2 and having a length of 35 amino acids or less.

According to one embodiment of the present disclosure, the peptide may entirely or partially include a cyclic structure.

The amino acid sequence of the cyclic peptide of the present disclosure may be an amino acid sequence of a VGLL1 peptide fragment containing serine at position 84 of a VGLL1 peptide sequence (Table 1) represented by SEQ ID NO: 67 and having a length of 35 amino acids or less.

**[Table 1]**

| |
|---|
| VGLL1 (SEQ ID NO: 67) |
| Met Glu Glu Met Lys Lys Thr Ala Ile Arg Leu Pro Lys Gly Lys Gln Lys Pro Ile Lys Thr Glu Trp Asn Ser Arg Cys Val Leu Phe Thr Tyr Phe Gln Gly Asp Ile Ser Ser Val Val Asp Glu His Phe Ser Arg Ala Leu Ser Asn Ile Lys Ser Pro Gln Glu Leu Thr Pro Ser Ser Gln Ser Glu Gly Val Met Leu Lys Asn Asp Asp Ser Met Ser Pro Asn Gln Trp Arg Tyr Ser Ser Pro Trp Thr Lys Pro Gln Pro Glu Val Pro Val Thr Asn Arg Ala Ala Asn Cys Asn Leu His Val Pro Gly Pro Met Ala Val Asn Gln Phe Ser Pro Ser Leu Ala Arg Arg Ala Ser Val Arg Pro Gly Glu Leu Trp His Phe Ser Ser Leu Ala Gly Thr Ser Ser Leu Glu Pro Gly Tyr Ser His Pro Phe Pro Ala Arg His Leu Val Pro Glu Pro Gln Pro Asp Gly Lys Arg Glu Pro Leu Leu Ser Leu Leu Gln Gln Asp Arg Cys Leu Ala Arg Pro Gln Glu Ser Ala Ala Arg Glu Asn Gly Asn Pro Gly Gln Ile Ala Gly Ser Thr Gly Leu Leu Phe Asn Leu Pro Pro Gly Ser Val His Tyr Lys Lys Leu Tyr Val Ser Arg Gly Ser Ala Ser Thr Ser Leu Pro Asn Glu Thr Leu Ser Glu Leu Glu Thr Pro Gly Lys Tyr Ser Leu Thr Pro Pro Asn His Trp Gly His Pro His Arg Tyr Leu Gln His Leu |

In the present disclosure, the cyclic peptide having the length of 35 amino acids or less may be 7 to 35 amino acids, preferably 7 to 25 amino acid residues, and more preferably 7 to 15 amino acids. Alternatively, the cyclic peptide includes an amino acid chain having a length of 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, or 35 amino acids, or any estimable range thereof.

In some embodiments, the cyclic peptide of the present disclosure may consist of any sequence having 35 or less consecutive amino acids in an amino acid sequence between positions 52 and 115 of the VGLL1 peptide sequence represented by SEQ ID NO: 67 and including serine at position 84.

For example, when the cyclic peptide according to the present disclosure includes serine at position 84 of the sequence in the terminal, the cyclic peptide may be a 30-amino acid sequence having the amino acid sequence at positions 55 to 84 of SEQ ID NO: 67. In addition, the cyclic peptide may be a 35-amino acid sequence having an amino acid sequence at positions 80 to 114 of SEQ ID NO: 67 including serine at position 80 of the sequence in the terminal.

Additionally, the cyclic peptide may be a sequence of any 35 or less amino acids including the serine sequence at position 84 in the amino acid sequence between positions 52 to 115 of SEQ ID NO: 67, and any sequence such that the total number of amino acid sequences is 35 or less based on the serine sequence at position 84 of SEQ ID NO: 67. For example, the cyclic peptide may be a sequence that further includes or consists of serine at position 84 in the amino acid sequence of the VGLL1 protein and 8 to 25 amino acids adjacent thereto, preferably 7 to 20 amino acids, and more preferably 6 to 15 amino acids.

Specifically, any sequence according to the present disclosure includes, for example, an amino acid sequence from any selected amino acid sequence at position 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, or 82 to any selected amino acid sequence at position 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114 or 115 of the VGLL1 peptide sequence represented by SEQ ID NO: 67, and any sequence may also be included in the peptide fragment within the scope of the present disclosure as long as its length satisfies the above-mentioned 35 or less.

Cytokine TGF-β induces phosphorylation of intracellular signaling proteins. When cancer cells are treated with TGF-β, VGLL1 phosphorylation may be induced. For example, a peptide fragment containing S84 of VGLL1 may inhibit the expression of MMP9 by competitively inhibiting the binding of VGLL1 and TEAD4. Amino acids that may be phosphorylated by TGF-β include serine, threonine, and tyrosine. Accordingly, it is not limited thereto, but in the amino acid sequence represented by SEQ ID NO: 67, it may be preferred that sequences at positions 54, 59, 61, 62, 64, 66, 74, 76, 82, 83, 84, and 96 are maintained without mutation.

Meanwhile, a preferred amino acid mutation at a substitution site may be performed based on similarity in polarity, charge, solubility, hydrophobicity, hydrophilicity, and/or amphipathic properties of the residues. For example, although not limited thereto, negatively charged amino acids may include aspartic acid and glutamic acid; positively charged amino acids may include lysine and arginine; and amino acids with uncharged polar head groups having similar hydrophilicity values may include leucine, isoleucine and valine; glycine and alanine; asparagine and glutamine; and serine, threonine, phenylalanine and tyrosine. The mutation may include conservative substitution. The 'conservative substitution' is a substitution that any amino acid is substituted with another amino acid having similar characteristics, and those skilled in the art may predict that the secondary structure and hydropathic nature (hydrophobic or hydrophilic nature) of the peptide had been substantially unchanged. In general, the following amino acid groups show conservative changes: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his.

For example, the conservative changes are exemplary amino acid substitutions that do not modify the biological activity, but are not limited thereto, but may include mutations at positions 85 and 87 of SEQ ID NO: 67. For example, in SEQ ID NO: 67, the conservative changes may be P85A and T85A mutations.

Although not limited thereto, the present disclosure may include the following amino acid mutations at the above-mentioned substitution positions: S->A, P->A, T->A, or Y->A.

The mutation may include non-conservative changes. In a preferred embodiment, the mutant peptide may differ from a native sequence by substitution, deletion or addition of five or fewer amino acids. The mutations may also be modified by, for example, deletion or addition of amino acids that have a minimal effect on the secondary structure and the hydropathic nature of the peptide.

In some cases, the mutation may be modification, such as phosphorylation, sulfation, acrylation, glycosylation, methylation, farnesylation, acetylation, and amidation.

According to one embodiment of the present disclosure, the cyclic structure includes cysteine (Cys)-cysteine (Cys) disulfide bonds or linkers.

The cyclic peptide of the present disclosure may have a cyclic structure due to the formation of a disulfide bond between the thiol groups of any pair of cysteines present in the peptide.

According to one embodiment of the present disclosure, the cyclic structure may be formed using amino acid mimetics rather than cysteine (Cys). The amino acid mimetics may have a structure containing a linker, and a cyclic structure linked by a linker may be formed through a chemical reaction of the amino acid mimetics.

According to one embodiment of the present disclosure, the peptide may consist of any one amino acid sequence selected from the group consisting of SEQ ID NO: 3, SEQ ID NO: 27, SEQ ID NO: 29, SEQ ID NO: 31, SEQ ID NO: 33, SEQ ID NO: 40, SEQ ID NO: 41, SEQ ID NO: 42, SEQ ID NO: 44, SEQ ID NO: 46, SEQ ID NO: 48 and SEQ ID NO: 50.

Yet another aspect of the present disclosure provides a pharmaceutical composition for preventing or treating cancer including the peptide.

The pharmaceutical composition of the present disclosure may include a pharmaceutically acceptable carrier. The pharmaceutically acceptable carrier included in the pharmaceutical composition of the present disclosure is generally used in preparation of drugs, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, mineral oil, and the like, but is not limited thereto. The pharmaceutical composition of the present disclosure may further include a lubricant, a wetting agent, a sweetening agent, a flavoring agent, an emulsion, a suspension, a preservative, and the like, in addition to the ingredients. Suitable pharmaceutically acceptable carriers and formulations are described in detail in the *Remington's Pharmaceutical Sciences* (22th ed., 2013).

According to one embodiment of the present disclosure, the pharmaceutical composition may be administered together with one or more substances that exhibit the activity of preventing or treating cancer.

In addition, the pharmaceutical composition according to one embodiment of the present disclosure may be used alone or in combination with methods using surgery, hormone therapy, drug therapy, and/or biological response regulators, for the prevention or treatment of cancer.

The pharmaceutical composition of the present disclosure may include various bases and/or additives necessary and suitable for the preparation of formulations thereof, and without departing from the range of reducing the effects thereof, may be prepared by further including known compounds, such as nonionic surfactants, silicone polymers, extenders, fragrances, preservatives, disinfectants, oxidation stabilizers, organic solvents, ionic or nonionic thickeners, softeners, antioxidants, free radical destroyers, opacifiers, stabilizers, emollients, silicone, α-hydroxy acid, anti-foaming agents, moisturizers, vitamins, insect repellents, flavorings, preservatives, surfactants, anti-inflammatory agents, substance P antagonists, fillers, polymers, propellants, alkalinizing or acidifying agents, or coloring agents.

A suitable dose of the pharmaceutical composition of the present disclosure may be variously prescribed by factors, such as a formulation method, an administration method, age, weight, and sex of a patient, a pathological condition, food, an administration time, an administration route, an excretion rate, and response susceptibility. The dose of the pharmaceutical composition of the present disclosure may be 0.001 to 1,000 mg/kg for adults.

The pharmaceutical composition of the present disclosure may be administered orally or parenterally.

The pharmaceutical composition of the present disclosure may be administered in various formulations when administered orally, and may be administered in the form of solid preparations such as pills, powders, granules, tablets, or capsules, and may further include various excipients, such as wetting agents, sweeteners, aromatics, preservatives, etc. Specifically, when the composition of the present disclosure is formulated in the form of powders, granules, tablets or capsules, the composition may further include suitable carriers, excipients and diluents commonly used in its preparation. The carriers, excipients and diluents may be used with, for example, lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate and/or mineral oil, but are not limited thereto. In addition, the composition may be prepared by including diluents or excipients, such as fillers, extenders, binders, wetting agents, disintegrants, and surfactants commonly used in formulation, and may further include lubricants such as magnesium stearate or talc, in addition to the excipients.

The pharmaceutical composition of the present disclosure may be administered in various formulations when administered parenterally. Solid formulations include tablets, pills, powders, granules, capsules, etc., and liquid formulations correspond to suspensions, oral solutions, emulsions, syrups, etc., and may include various excipients, such as wetting agents, sweeteners, fragrances, and preservatives, in addition to commonly used simple diluents such as water, liquid, and paraffin. Specifically, the formulations for parenteral administration may include a sterile aqueous solution, a nonaqueous solution, a suspension, an emulsion, and a lyophilizing agent. As the nonaqueous solution and the suspension, propylene glycol, polyethylene glycol, vegetable oil such as olive oil, injectable ester such as ethyl oleate, and the like may be used. In addition, calcium or vitamin D3 may be added to improve the efficacy of therapeutic agents.

These compositions may be presented in unit-dose (single-dose) or multi-dose (several-dose) containers, such as sealed ampoules and vials, and may be stored under freeze-dried conditions requiring only the addition of a sterile liquid carrier, such as injections, immediately before use. Immediate preparations and suspensions may be prepared from sterile powders, granules and tablets.

According to one embodiment of the present disclosure, the peptide may be a fusion peptide that is linked to a peptide having a length of 20 amino acids or less, including an amino acid sequence in which an amino acid sequence represented by the following Formula I is linked to an N-terminus or C-terminus of an amino acid sequence represented by SEQ ID NO: 7:

[Formula I] Xaa₁-Xaa₂-Xaa₃-Xaa₄-Xaa₅

in Formula, Xaa₁ is an amino acid selected from the group consisting of Arg, Lys, Ala, Val, Ile, Leu, Met, Phe, Tyr and Trp, Xaa₂ is an amino acid selected from the group consisting of Arg, Lys and His, Xaa₃ is an amino acid of Arg or Leu, Xaa₄ is an amino acid selected from the group consisting of Arg, Lys, His, Asp, Glu, Ser, Thr, Asn and Gln, and Xaa₅ is an amino acid selected from the group consisting of Arg, Lys and Val.

According to one embodiment of the present disclosure, the peptide may consist of any one amino acid sequence selected from the group consisting of SEQ ID NO: 27, SEQ ID NO: 29, SEQ ID NO: 31, SEQ ID NO: 33, SEQ ID NO: 40, SEQ ID NO: 41, SEQ ID NO: 42, SEQ ID NO: 44, SEQ ID NO: 46, SEQ ID NO: 48 and SEQ ID NO: 50.

According to one embodiment of the present disclosure, the cancer may be any one cancer selected from the group consisting of stomach cancer, liver cancer, colon cancer, lung cancer, breast cancer, pancreatic cancer, head and neck squamous cell carcinoma, esophageal cancer, ovarian cancer, cervical cancer, endometrial cancer, mesothelioma, melanoma, sarcoma, osteosarcoma, liposarcoma, thyroid cancer, desmoid tumor, and blood cancer.

### [Mode for Carrying Out the Invention]

Hereinafter, the present disclosure will be described in more detail with reference to one or more Examples. However, these Examples are only illustrative of the present disclosure, and the scope of the present disclosure is not limited to these Examples.

### Example 1. Confirmation of increased cancer cell inhibitory effect according to cyclization of anticancer peptide fragment

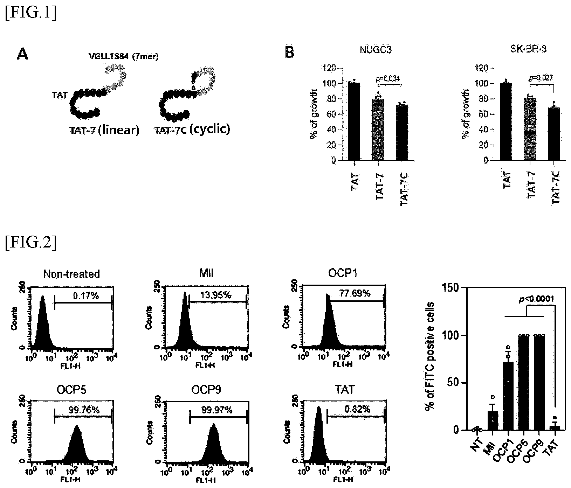

To determine whether the stability and a cancer cell inhibitory effect increased due to cyclization of an anticancer peptide fragment, the cancer cell inhibitory effect was analyzed in a linear anticancer peptide (7 in Table 2) or a cyclic anticancer peptide (CC in Table 2) cyclized by a disulfide bond between cysteine (C) linked to TAT, an HIV-derived cell-penetrating peptide (CPP) (Con in Table 2) (FIG. 1A).

**[Table 2]**

| Name | Amino acid sequence | SEQ ID NO: | Comparison |
|---|---|---|---|
| linear peptide | YSSPWTK | SEQ ID NO: 2 | Linear anticancer peptide |
| cyclic peptide | CYSSPWTKC | SEQ ID NO: 3 | Cyclic anticancer peptide |
| Con | YGRKKRRQRRR | SEQ ID NO: 4 | TAT (CPP) |
| 7 | YGRKKRRQRRR- YSSPWTK | SEQ ID NO: 5 | TAT-linear anticancer peptide |
| CC | YGRKKRRQRRR- CYSSPWTKC | SEQ ID NO: 6 | TAT-cyclic anticancer peptide |

Specifically, in order to confirm cell growth ability, a stomach cancer cell line NUGC3 and a breast cancer cell line SK-BR-3 were each subcultured in a 96-well plate, and the next day, peptide fragments Con, 7, and CC of Table 2 were treated with 100 µM each to the cell lines. After 72 hours, the cells were immobilized with a 10% trichloroacetic acid solution, and then stained the cell with a 0.4% sulforhodamine B solution for 1 hour, washed with acetic acid, and dried. Thereafter, the dried cells were dissolved in a 10 mM Tris solution and absorbance was measured at 510 nm using an ELISA plate reader.

As a result, it was confirmed that the cell growth inhibitory effect of the cyclic anticancer peptide (SEQ ID NO: 6) was significantly superior to that of the linear anticancer peptide (SEQ ID NO: 5) (FIG.1B).

### Example 2. Investigation of synthesis and cell permeability of various novel cell-penetrating peptides

First, in order to develop cancer therapeutic peptides with improved anticancer effects, it was attempted to develop novel cell-penetrating peptides to increase the intracellular permeability of the anticancer peptide. Therefore, novel cell-penetrating peptides having various structures were synthesized (Table 3).

**[Table 3]**

| Name | Amino acid sequence | SEQ ID NO: |
|---|---|---|
| SII | SIIFRIF | SEQ ID NO: 7 |
| OCP1 | SIIFRIFLHLQK | SEQ ID NO: 8 |
| OCP2 | SIIFRIFAHLQK | SEQ ID NO: 9 |
| OCP3 | SIIFRIFLHLKK | SEQ ID NO: 10 |
| OCP4 | SIIFRIFAHLKK | SEQ ID NO: 11 |
| OCP5 | SIIFRIFLHLQKRRR | SEQ ID NO: 12 |
| OCP6 | SIIFRIFAHLQKRRR | SEQ ID NO: 13 |
| OCP7 | SIIFRIFLHLKKRRR | SEQ ID NO: 14 |
| OCP8 | SIIFRIFAHLKKRRR | SEQ ID NO: 15 |
| OCP9 | SIIFRIFRRRRR | SEQ ID NO: 16 |
| OCP10 | SIIFRIFRRRRRGD | SEQ ID NO: 17 |
| OCP11 | RRRRRSIIFRIF | SEQ ID NO: 18 |
| OCP12 | RRRRRSIIFRGD | SEQ ID NO: 19 |
| OCP13 | RRRRRGDSIIF | SEQ ID NO: 20 |
| OCP14 | RRRRRGDSIIFRIF | SEQ ID NO: 21 |
| OCP15 | KKKKRKVRGDSI | SEQ ID NO: 22 |
| OCP16 | KKKKRKVSIIFIRGD | SEQ ID NO: 23 |
| OCP17 | KKKKRKVSIRGD | SEQ ID NO: 24 |
| OCP18 | KKKKRKVSIIFRIF | SEQ ID NO: 25 |

To quantitatively measure the cell permeability of the novel synthesized cell-penetrating peptides, 1.5 or 2.5 µM FITC-conjugated peptides were administered to the cell line NUGC3 or the normal cell line WI38, and after 3 hours, the amounts of FITC present in the cells were measured by FACS.

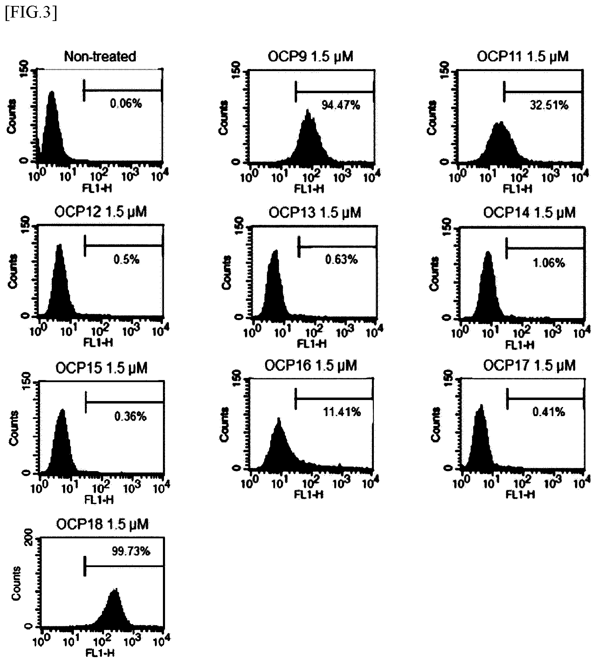

As a result, it was confirmed that the intracellular permeability of peptide fragments OCP5 and OCP9 containing an SIIFRIF amino acid sequence (SII) and three (3R) or five (5R) arginines was significantly increased compared to TAT, a conventional CPP (FIGS. 2 and 3). In addition, it was confirmed that the intracellular permeability of a peptide OCP18 (SEQ ID NO: 25) was significantly increased compared to the conventional TAT.

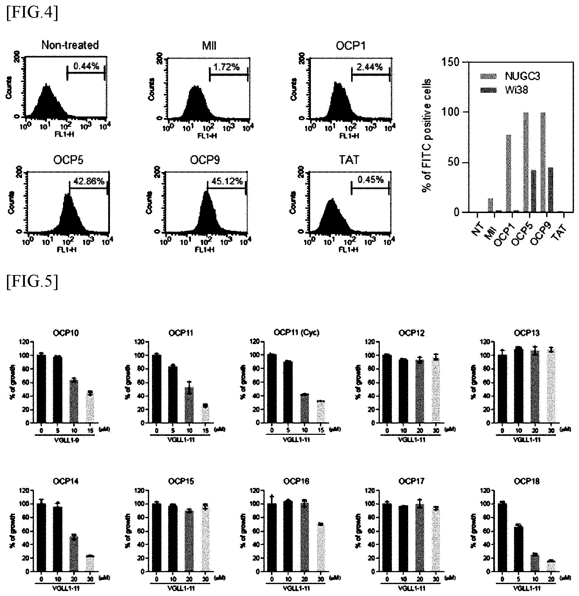

On the other hand, it was shown that the permeability of the novel peptides to the normal cell line WI38 was reduced by 40% to 70% for each OCP5 compared to the stomach cancer cell line NUGC3 (FIG. 4), but it was confirmed that the peptide fragments containing the SIIFRIF amino acid sequence and 3 or 5 arginines had improved cell permeability specifically for cancer cells.

### Example 3. Confirmation of cancer cell growth inhibitory effects of cancer therapeutic peptides bound with novel cell-penetrating peptides and anticancer peptide

### 3-1. Comparison of cancer cell growth inhibitory effect of cancer therapeutic peptides according to cell-penetrating peptides

In order to compare the cancer cell growth inhibitory effects of cancer therapeutic peptides according to cell-penetrating peptides, the stomach cancer cell line NUGC3 was treated with 10 µM each of cancer therapeutic peptide fragments in Table 4, and the cancer cell growth inhibitory effects were screened in the same manner as in Example 1.

**[Table 4]**

| Name | Amino acid sequence | SEQ ID NO: |
|---|---|---|
| OCP1-VGLL1S84-15 | SIIFRIFLHLQK- PNQWRYSSPWTKPQP | SEQ ID NO: 26 |
| OCP1-VGLL1S84-15C | SIIFRIFLHLQK- CPNQWRYSSPWTKPQPC | SEQ ID NO: 27 |
| OCP5-VGLL1S84-15 | SIIFRIFLHLQK- PNQWRYSSPWTKPQP-RRR | SEQ ID NO: 28 |
| OCP5-VGLL1S84-15C | SIIFRIFLHLQK- CPNQWRYSSPWTKPQPC-RRR | SEQ ID NO: 29 |
| OCP1-VGLL1S84-7 | SIIFRIFLHLQK-YSSPWTK | SEQ ID NO: 30 |
| OCP1-VGLL1S84-7C | SIIFRIFLHLQK-CYSSPWTKC | SEQ ID NO: 31 |
| OCP5-VGLL1S84-7 | SIIFRIFLHLQK-YSSPWTK-RRR | SEQ ID NO: 32 |
| OCP5-VGLL1S84-7C | SIIFRIFLHLQK-CYSSPWTKC-RRR | SEQ ID NO: 33 |
| OCP2-VGLL1S84-7 | SIIFRIFAHLQK-YSSPWTK | SEQ ID NO: 34 |
| OCP2-VGLL1S84-7C | SIIFRIFAHLQK-CYSSPWTKC | SEQ ID NO: 35 |
| OCP3-VGLL1S84-7 | SIIFRIFLHLKK-YS SPWTK | SEQ ID NO: 36 |
| OCP3-VGLL1S84-7C | SIIFRIFLHLKK-CYSSPWTKC | SEQ ID NO: 37 |
| OCP4-VGLL1S84-7 | SIIFRIFAHLKK-YS SPWTK | SEQ ID NO: 38 |
| OCP4-VGLL1S84-7C | SIIFRIFAHLKK-CYSSPWTKC | SEQ ID NO: 39 |
| OCP5-VGLL1S84-7CC | CSIIFRIFLHLQK-YSSPWTK-RRRC | SEQ ID NO: 40 |
| OCP5-VGLL1S84-9C | SIIFRIFLHLQK-CWRYSSPWTKC- RRR | SEQ ID NO: 41 |
| OCP5-VGLL1S84-11C | SIIFRIFLHLQK-CNQWRYSSPWTKC-RRR | SEQ ID NO: 42 |
| OCP9-VGLL1S84-7 | SIIFRIFRRRRR-YSSPWTK | SEQ ID NO: 43 |
| OCP9-VGLL1S84-7CC | CSIIFRIFRRRRR-YSSPWTKC | SEQ ID NO: 44 |
| OCP9-VGLL1S84-9 | SIIFRIFRRRRR-WRYS SPWTK | SEQ ID NO: 45 |
| OCP9-VGLL1S84-9CC | CSIIFRIFRRRRR-WRYSSPWTKC | SEQ ID NO: 46 |
| OCP9-VGLL1S84-11 | SIIFRIFRRRRR-NQWRYSSPWTK | SEQ ID NO: 47 |
| OCP9-VGLL1S84-11CC | CSIIFRIFRRRR- NQWRYSSPWTKC | SEQ ID NO: 48 |
| OCP9-VGLL1S84-15 | SIIFRIFRRRR- PNQWRYSSPWTKPQP | SEQ ID NO: 49 |
| OCP9-VGLL1S84-15CC | CSIIFRIFRRRRR- PNQWRYSSPWTKPQPC | SEQ ID NO: 50 |
| OCP10-VGLL1S84-9 | SIIFRIFRRRRRGD-WRYSSPWTK | SEQ ID NO: 51 |
| OCP11-VGLL1S84-11 | RRRRRSIIFRIF-NQWRYSSPWTK | SEQ ID NO: 52 |
| OCP11-VGLL1S84-11CC | CRRRRRSIIFRIF- NQWRYSSPWTKC | SEQ ID NO: 53 |
| OCP12-VGLL1S84-11 | RRRRRSIIFRGD-NQWRYSSPWTK | SEQ ID NO: 54 |
| OCP13-VGLL1S84-11 | RRRRRGDSIIF-NQWRYSSPWTK | SEQ ID NO: 55 |
| OCP14-VGLL1S84-11 | RRRRRGDSIIFRIF- NQWRYSSPWTK | SEQ ID NO: 56 |
| OCP15-VGLL1S84-11 | KKKKRKVRGDSI- NQWRYSSPWTK | SEQ ID NO: 57 |
| OCP16-VGLL1S84-11 | KKKKRKVSIIFIRGD- NQWRYSSPWTK | SEQ ID NO: 58 |
| OCP17-VGLL1S84-11 | KKKKRKVSIRGD- NQWRYSSPWTK | SEQ ID NO: 59 |
| OCP18-VGLL1S84-11 | KKKKRKVSIIFRIF- NQWRYSSPWTK | SEQ ID NO: 60 |

As a result, as shown in FIG. 5, it was confirmed that cancer therapeutic peptide fragments, excluding OCP12, OCP13, OCP15, OCP16, and OCP17, had increased cell permeability to exhibit the cancer cell growth inhibitory effects. In addition, it was confirmed that the cell permeability was increased by OCP1 (SEQ ID NO: 8) and OCP5 (SEQ ID NO: 12), and then cancer therapeutic peptide fragments (SEQ ID NO: 31 and SEQ ID NO: 33) had a significant cancer cell growth inhibitory effect even in a 1/10 level of existing used concentration. In particular, in the case of OCP5, it was confirmed that the cancer cell growth inhibitory effect increased due to an increase in water solubility.

Meanwhile, from Example 1, it was confirmed that the cyclic anticancer peptide had a superior cancer cell growth inhibitory effect to the linear anticancer peptide. Therefore, novel CPPs were synthesized and selected to improve the anticancer effect of the cyclic anticancer peptides.

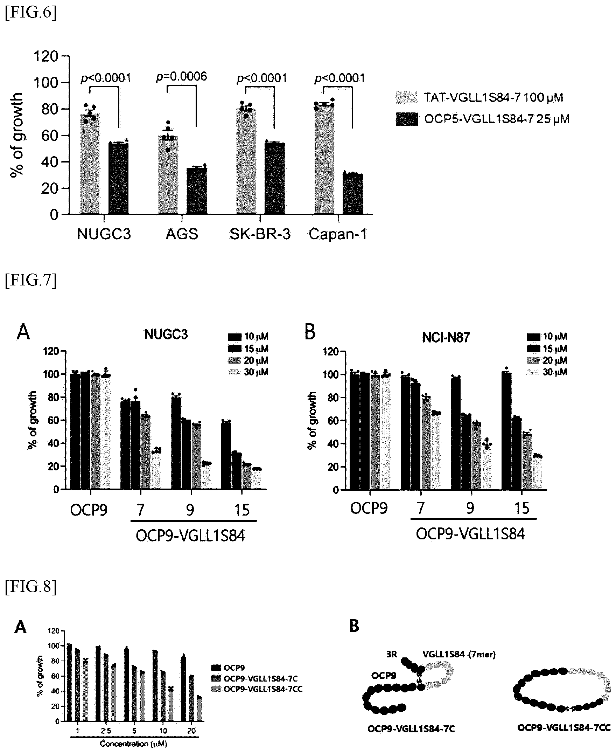

Specifically, in order to confirm the cancer cell inhibition enhancing effect by enhancing the cell permeability of OCP5 among the novel synthesized CPPs, a cancer therapeutic peptide fragment represented by SEQ ID NO: 6 including the existing TAT and a cancer therapeutic peptide fragment represented by SEQ ID NO: 31 including the novel synthesized OCP5 were used. The stomach cancer cell lines NUGC3 and AGS, the breast cancer cell line SK-BR-3, and the pancreatic cancer cell line Capan-1 were treated with 25 µM SEQ ID NO: 6 and 100 µM SEQ ID NO: 31 for 72 hours, respectively, and the cancer cell growth inhibitory effects were compared in the same manner as in Example 1. As a result, the anticancer peptide fragment including the novel synthesized OCP5 showed the cancer cell growth inhibitory effect 8 to 10 times greater than the existing TAT (FIG. 6). Through this, it was confirmed that the novel CPP may be applied more effectively than the existing TAT to inhibit the cancer cell growth by the anticancer peptides.

### 3-3. Comparison of cancer cell growth inhibitory effects according to anticancer peptide fragment length

To analyze the cancer cell growth inhibitory effect according to a length of the anticancer peptide fragment, OCP9-VGLL1S84-7 (SEQ ID NO: 43), OCP9-VGLL1S84-9CC (SEQ ID NO: 46), and OCP9-VGLL1S84-15 (SEQ ID NO: 49) were treated for each concentration to analyze the growth inhibitory effects of stomach cancer cell lines NUGC3 and NCI-N87. When stomach cancer cells were treated with cancer therapeutic peptides, the cancer cell growth inhibitory efficacy was shown in a concentration-dependent manner. In addition, it was confirmed that the growth inhibitory effect on the stomach cancer cell lines increased as the length of the anticancer peptide fragment increased (FIG. 7).

### 3-4. Comparison of cancer cell growth inhibitory effects according to cyclic anticancer peptide

Using a cancer therapeutic peptide fragment in which a linear or cyclic anticancer peptide fragment was linked to the novel CPP, the cancer cell growth inhibitory effect of the cyclic anticancer peptide was investigated.

Specifically, the cancer cell growth inhibitory effect according to partial or entire cyclization of the anticancer peptide fragment was analyzed. The stomach cancer cell line NUGC3 was treated with a CPP fragment OCP9 (SEQ ID NO: 16), a partially cyclized sequence OCP9-VGLL1S84-7C (SEQ ID NO: 33), or a sequence OCP9-VGLL1S84-7CC (SEQ ID NO: 40) including an entirely cyclized anticancer peptide at different concentrations of 1, 2.5, 5, 10, and 20 µM, respectively, and the cancer cell growth inhibitory effects were compared in the same manner as in Example 1.

As a result, it was confirmed that when the VGLL1 peptide was entirely cyclized, the cancer cell growth inhibitory effect was increased compared to the non-cyclized or partially cyclized peptide (FIG. 8).

### Example 4. Analysis of stability of cancer therapeutic peptide fragments on normal cells

To analyze the cytotoxicity of cell therapeutic peptide fragments on normal cell lines, lung normal cell lines WI38 and CCD39 were treated with OCP9 (SEQ ID NO: 16), OCP9-VGLL1S84-7CC (SEQ ID NO: 44), OCP9-VGLL1S84-9CC (SEQ ID NO: 46) and OCP9-VGLL1S84-11CC (SEQ ID NO: 48) at 30 µM and 60 µM, respectively, and the growth degrees of the cell lines were evaluated.

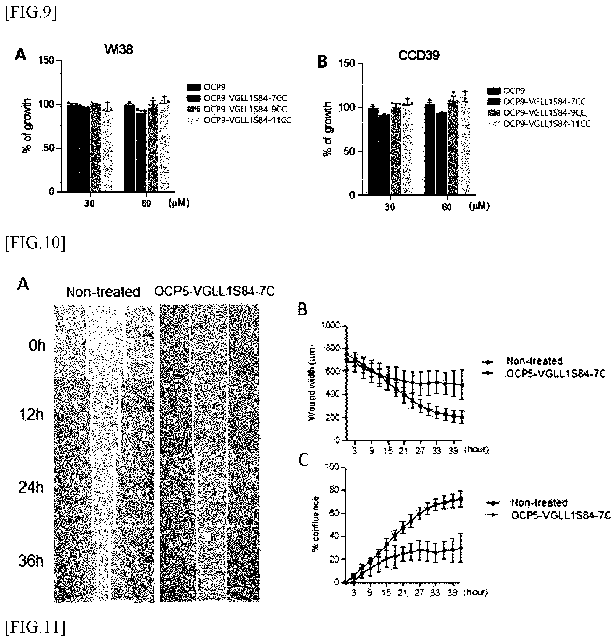

As a result, OCP9-VGLL1S84-7CC, OCP9-VGLL1S84-9CC, and OCP9-VGLL1S84-11CC were confirmed not to exhibit the growth inhibitory or abnormal effect on the normal lung cell lines and thus evaluated to have no cytotoxicity (FIG. 9).

### Example 5. Analysis of metastasis inhibitory effect of cancer therapeutic peptide fragments

### 5-1. Cancer cell migration inhibitory effect of anticancer therapeutic peptide

In order to analyze the possibility of metastasis inhibitory efficacy of stomach cancer cells by anticancer therapeutic peptides, a wound healing assay was performed to evaluate a migration inhibitory effect of stomach cancer cells.

Specifically, 5 × 10⁴ stomach cancer cell lines NUGC3 were subcultured in a 24-well plate, and then the next day, scratches were made at the center of the cells, and 10 µM OCP5-VGLL1S84-7C (SEQ ID NO: 33) was mixed into the cell culture medium and administered. Real-time cell images were acquired through Cytation5 (Biotek), and the degree of cancer cell migration was analyzed by calculating the average area (wound width, B) of the scratch area and the degree (%Confluence, C) to which cells were filled.

As a result, it was confirmed that the migration of cancer cells treated with OCP5-VGLL1S84-7C was inhibited (FIG. 10).

### 5-2. Cancer cell invasion inhibitory effect of anticancer therapeutic peptide

Boyden chamber assay was performed to analyze the invasion inhibition possibility of cancer therapeutic peptides.

Specifically, a Boyden chamber transwell (Corning Life Sciences, #353097) was inserted into a 24-well plate, and then 10⁶ stomach cancer cell lines (AGS), OCP9 (SEQ ID NO: 16), OCP9-VGLL1S84-7CC (SEQ ID NO: 44), OCP9-VGLL1S84-9CC (SEQ ID NO: 46), and OCP9-VGLL1S84-11CC (SEQ ID NO: 48) were mixed at each 10 µM in the cell culture medium and administered to a transwell. After 72 hours, the transwell was removed, and the cells at the bottom of the 24-well plate were immobilized with 4% paraformaldehyde and stained with a 0.5% crystal violet solution. Cancer cell photographs were obtained using a Nikon TS2 microscope. In the graph, after three repeated experiments, the cells were counted and the relative value was calculated based on an OCP9-administered group set as 100%.

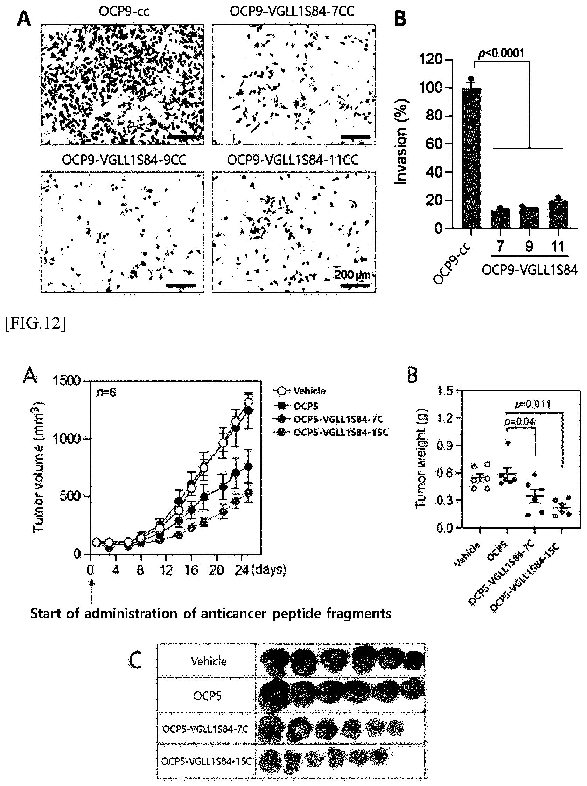

As a result, it was confirmed that the invasion of the stomach cancer cell lines was inhibited by entire cyclic anticancer peptide fragments OCP9-VGLL1S84-7CC, OCP9-VGLL1S84-9CC, and OCP9-VGLL1S84-11CC (FIG. 11).

### Example 6. Evaluation of antitumor efficacy of cancer therapeutic peptide fragments according to mouse xenograft experiment

### Example 6-1. Investigation of in vivo tumor formation inhibitory efficacy of cancer therapeutic peptide fragments

A mouse xenograft experiment was performed to analyze a tumor formation inhibitory effect of cancer therapeutic peptide fragments.

Specifically, 5 × 10⁶ stomach cancer cell lines NUGC3 were transplanted by subcutaneous injection into a flank site of a 6-week-old female Balb-c/nude mouse, and after one week (average tumor size 100 mm³), Vehicle (0.9% saline), OCP5 (SEQ ID NO: 12), OCP5-VGLL1S84-15C (SEQ ID NO: 29), or OCP5-VGLL1S84-7C (SEQ ID NO: 33) was each administered intraperitoneally at 10 mg/kg once a day. Tumor size was measured with a digital caliper at 2-day intervals from the start of drug administration, and the tumor size (tumor size (mm³) = long axis × short axis²) was calculated. The experiment was terminated when the average tumor size of the OCP5-administered group was 1,000 mm³, and then the tumor was extracted, and the tumor weight was measured.

In FIG. 11, the tumor size (FIG. 12A), the weight of the extracted tumor (FIG. 12B), and a photograph of the extracted tumor (FIG. 12C) during the administration period of the cancer therapeutic peptides were shown. It was shown that the mouse body weight was not affected in all the experimental groups. In addition, through analysis of the experimental results, it was confirmed that the OCP5-administered group did not affect the tumor formation compared to the vehicle group, but the OCP5-VGLL1S84-7C and OCP5-VGLL1S84-15C-administered groups showed tumor formation inhibitory effects of 45% and 63%, respectively, compared to the vehicle group (FIG. 12).

### Example 6-2. Analysis of quantitative changes in MMP9 in tumors administered with cancer therapeutic peptide fragments

VGLL1 phosphorylated by TGF-β increased the expression of a target protein MMP9 through interaction with TEAD4 to induce the invasion and metastasis of cancer. Accordingly, a relation between VGLL1S84 anticancer efficacy and MMP9 was intended to be confirmed by analyzing the expression level of MMP9 in mouse tumor tissue administered with the cancer therapeutic peptide fragments of the present disclosure.

After completion of the in vivo xenograft experiment in Example 6-1, changes in the protein expression level of MMP9 were measured in the extracted tumor tissue using immunohistochemical staining and Western blot methods.

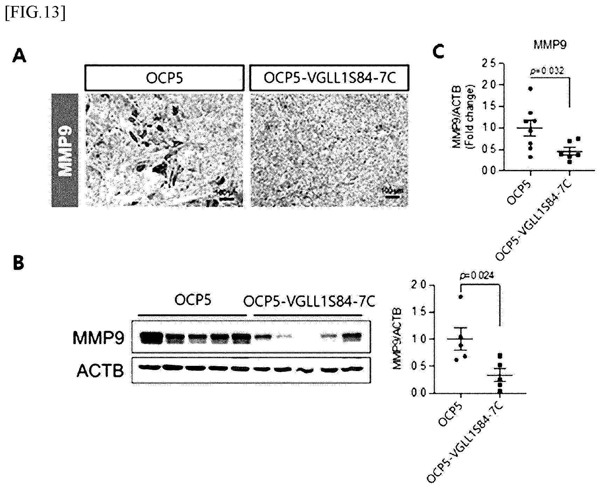

The immunohistochemical staining was performed by the following method. The extracted tumor tissue was immobilized with 4% paraformaldehyde and embedded in paraffin, and then the tissue was sectioned at 4 µm thickness and attached to a glass slide. The tissue was hydrated with a xylene-ethanol reaction, and then reacted with an MMP9 antibody (1 : 100) at 4°C overnight. The next day, the tissue sections were washed with PBS and reacted with a secondary antibody HRP-conjugated anti-rabbit antibody (1 : 100) at room temperature for 1 hour, and then the antibody was washed with PBS, and the reacted MMP9 was searched through the reaction by a DAB substrate kit (Vecotr Laboratories, #SK-4100). After DAB washing, nuclear staining was performed with a hematoxylin solution, and the tissue was emulsified through an ethanol-xylene reaction and then mounted. Tissue photographs were obtained using a Nikon TS2 microscope. As a result, it was confirmed that in the groups administered with the cancer therapeutic peptide fragments OCP5- VGLL 1 S84-7C and OCP5-VGLL1S84-15C, the amount of MMP9 protein in the tumor was significantly reduced (FIG. 13A).

In addition, the amount of MMP9 protein in the tumor tissue was analyzed by Western blot experiment. The protein amount in the sample was quantified using a BCA assay method, and 20 µg of each sample was loaded, electrophoresed on SDS-PAGE, and then transferred to a nitrocellulose membrane. The membrane was blocked in a 5% skim milk solution for 1 hour, and then placed in MMP9 (1 : 1000, Abcam #ab76003) or ACTB (1 : 10,000, SantaCruz #sc-47778) antibody solution and reacted overnight at 4°C. The next day, the primary antibody was removed, the membrane was washed with a TBST buffer, and a HRP-conjugated anti-mouse or anti-rabbit secondary antibody was diluted at 1 : 10,000 in the 5% skim milk solution, and then reacted with the membrane at room temperature for 1 hour. The blot reacted with a SuperSignalTM West Pico Chemiluminescent substrate (ThermoFisher #34580) and analyzed on the ChemiDoc XRS+ System (Bio-Rad Laboratories, #1708265). Through the Western blot experiment, it was confirmed that the amount of MMP9 was also reduced in tumor tissues administered with the cancer therapeutic peptide fragments OCP5-VGLL1S84-7C and OCP5-VGLL1S84-15C (FIG. 12B).

In addition, the mRNA amount of MMP9 in the extracted tumor tissue in Example 6-1 was analyzed through a qRT-PCR experiment. The tissue was lyzed, and then total RNA was isolated using a NucleoSpin RNA kit (Macherey-Nagel, #740955.250). The cDNA was synthesized from total RNA of 500 ng of each sample using a SuperiorScript III cDNA synthesis kit (Enzynomics, #EZ405M). 1 µM an MMP9 or ACTB primer pair of Table 5, 1 ng of synthesized cDNA, and Sybr green (Enzynomics, #RT432M) were added, and real-time PCR was performed on CFX connect (Bio-Rad Laboratories, #1855201). All samples were PCR synthesized in duplicate, and MMP9 PCR values were corrected with ACTB PCR values and calculated (FIG. 13C).

**[Table 5]**

| Primer name | Nucleotide sequence (5' → 3') | SEQ ID NO: |
|---|---|---|
| MMP1_foward | ATGAAGCAGCCCAGATGTGGAG | SEQ ID NO: 61 |
| MMP1_reverse | TGGTCCACATCTGCTCTTGGCA | SEQ ID NO: 62 |
| MMP9_foward | GGGACGCAGACATCGTCATC | SEQ ID NO: 63 |
| MMP9_reverse | TCGTCATCGTCGAAATGGGC | SEQ ID NO: 64 |
| ACTB_foward | CACCATTGGCAATGAGCGGTTC | SEQ ID NO: 65 |
| ACTB_reverse | AGGTCTTTGCGGATGTCCACGT | SEQ ID NO: 66 |

In conclusion, it was shown that the protein expression (FIGS. 13A and 13B) and the mRNA amount (FIG. 13C) of MMP9 in the tumor tissue administered with the cancer therapeutic peptide fragments were significantly reduced compared to the vehicle and the OCP-administered groups. Therefore, it was confirmed that the cancer therapeutic peptide fragments of the present disclosure were able to be effectively applied to cancer treatment.

### Example 7. Analysis of growth inhibitory efficacy of cancer therapeutic peptides on various cancer cells

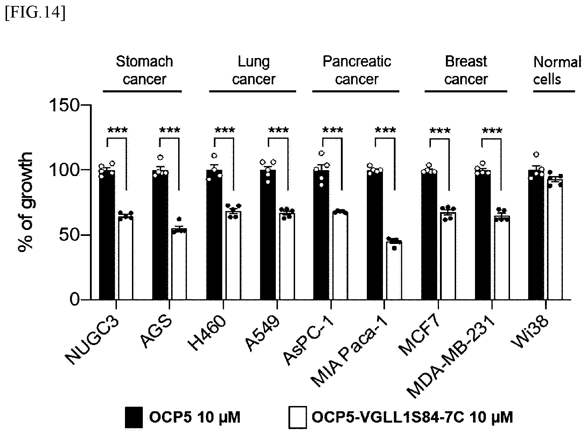

It was investigated whether the cancer therapeutic peptide synthesized in this study could be used to treat various cancers other than stomach cancer. For this purpose, the cancer therapeutic peptides OCP and OCP5-VGLL1S84-7C were treated in stomach cancer cell lines NUGC2 and AGS, lung cancer cell lines H460 and A549, pancreatic cancer cell lines AsPC-1 and MIAPaca-1, and breast cancer cell lines MCF7 and MDA-MB-231 at concentrations of 10 µM. Thereafter, as a result of analysis in the same manner as in Example 1, the growth inhibitory efficacy of OCP5-VGLL1S84-7C cancer cells was confirmed compared to the control OCP5 (FIG. 14).

The present disclosure has been described above with reference to the embodiments thereof. It will be understood to those skilled in the art that the present disclosure may be implemented as modified forms without departing from an essential characteristic of the present disclosure. Therefore, the disclosed embodiments should be considered in an illustrative viewpoint rather than a restrictive viewpoint. The scope of the present disclosure is defined by the appended claims rather than by the foregoing description, and all differences within the scope of equivalents thereof should be construed as being included in the present disclosure.

## Claims

1. A peptide having a length of 20 amino acids or less, comprising an amino acid sequence in which an amino acid sequence represented by the following Formula I is linked to an N-terminus or C-terminus of an amino acid sequence represented by SEQ ID NO: 7:
[Formula I] Xaa₁-Xaa₂-Xaa₃-Xaa₄-Xaa₅
wherein,
Xaa₁ is an amino acid selected from the group consisting of Arg, Lys, Ala, Val, Ile, Leu, Met, Phe, Tyr and Trp,
Xaa₂ is an amino acid selected from the group consisting of Arg, Lys and His,
Xaa₃ is an amino acid of Arg or Leu,
Xaa₄ is an amino acid selected from the group consisting of Arg, Lys, His, Asp, Glu, Ser, Thr, Asn and Gln, and
Xaa₅ is an amino acid selected from the group consisting of Arg, Lys and Val.

2. The peptide of claim 1, wherein 1 to 10 arginines (Arg) or lysines (Lys) are further linked to the N-terminus or C-terminus of the peptide.

3. The peptide of claim 2, wherein the peptide consists of an amino acid sequence represented by SEQ ID NO: 12 or SEQ ID NO: 25.

4. A cell penetrating composition comprising the peptide of claim 1.

5. A peptide comprising an amino acid sequence represented by SEQ ID NO: 2 and having a length of 35 amino acids or less.

6. The peptide of claim 5, wherein the peptide includes entirely or partially a cyclic structure.

7. The peptide of claim 6, wherein the cyclic structure includes cysteine (Cys)-cysteine (Cys) disulfide bonds or linkers.

8. The peptide of claim 6, wherein the peptide consists of any one amino acid sequence selected from the group consisting of SEQ ID NO: 3, SEQ ID NO: 27, SEQ ID NO: 29, SEQ ID NO: 31, SEQ ID NO: 33, SEQ ID NO: 40, SEQ ID NO: 41, SEQ ID NO: 42, SEQ ID NO: 44, SEQ ID NO: 46, SEQ ID NO: 48 and SEQ ID NO: 50.

9. A pharmaceutical composition for preventing or treating cancer comprising the peptide of claim 5.

10. The pharmaceutical composition for preventing or treating cancer of claim 9, wherein the peptide is a fusion peptide that is linked to a peptide having a length of 20 amino acids or less, including an amino acid sequence in which an amino acid sequence represented by the following Formula I is linked to an N-terminus or C-terminus of an amino acid sequence represented by SEQ ID NO: 7:
[Formula I] Xaa₁-Xaa₂-Xaa₃-Xaa₄-Xaa₅
wherein,
Xaa₁ is an amino acid selected from the group consisting of Arg, Lys, Ala, Val, Ile, Leu, Met, Phe, Tyr and Trp,
Xaa₂ is an amino acid selected from the group consisting of Arg, Lys and His,
Xaa₃ is an amino acid of Arg or Leu,
Xaa₄ is an amino acid selected from the group consisting of Arg, Lys, His, Asp, Glu, Ser, Thr, Asn and Gln, and
Xaa₅s is an amino acid selected from the group consisting of Arg, Lys and Val.

11. The pharmaceutical composition for preventing or treating cancer of claim 9, wherein the peptide consists of any one amino acid sequence selected from the group consisting of SEQ ID NO: 27, SEQ ID NO: 29, SEQ ID NO: 31, SEQ ID NO: 33, SEQ ID NO: 40, SEQ ID NO: 41, SEQ ID NO: 42, SEQ ID NO: 44, SEQ ID NO: 46, SEQ ID NO: 48 and SEQ ID NO: 50.

12. The pharmaceutical composition for preventing or treating cancer of claim 9, wherein the cancer is any one cancer selected from the group consisting of stomach cancer, liver cancer, colon cancer, lung cancer, breast cancer, pancreatic cancer, head and neck squamous cell carcinoma, esophageal cancer, ovarian cancer, cervical cancer, endometrial cancer, mesothelioma, melanoma, sarcoma, osteosarcoma, liposarcoma, thyroid cancer, desmoid tumor, and blood cancer.
